(19) Europäisches Patentamt
European Patent Office
Office européen des brevets

(11) Publication number : **0 403 249 B1**

(12) # EUROPEAN PATENT SPECIFICATION

(45) Date of publication of patent specification : **27.04.94 Bulletin 94/17**

(51) Int. Cl.$^5$ : **C07C 317/46,** C07C 323/62, C07C 323/56, C07D 311/24, A61K 31/19, A61K 31/215, A61K 31/35

(21) Application number : **90306438.4**

(22) Date of filing : **13.06.90**

(54) **Leukotriene antagonists.**

(30) Priority : **14.06.89 US 366046**

(43) Date of publication of application : **19.12.90 Bulletin 90/51**

(45) Publication of the grant of the patent : **27.04.94 Bulletin 94/17**

(84) Designated Contracting States : **AT BE CH DE DK ES FR GB GR IT LI LU NL SE**

(56) References cited :
**EP-A- 0 296 732**
**EP-A- 0 313 697**

(73) Proprietor : **SMITHKLINE BEECHAM CORPORATION**
**P.O. Box 7929 1 Franklin Plaza**
**Philadelphia Pennsylvania 19101 (US)**

(72) Inventor : **Frazee, James Simpson**
**5 Buffalo Run**
**Sewell, New Jersey 08080 (US)**
Inventor : **Gleason, John Gerald**
**8 Heron Hill Drive**
**Downingtown, Pennsylvania 19355 (US)**
Inventor : **Hall, Ralph Floyd**
**1311 Prospect Hill Road**
**Villanova, Pennsylvania 19085 (US)**

(74) Representative : **Giddings, Peter John, Dr. et al**
**SmithKline Beecham, Corporate Patents, Mundells**
**Welwyn Garden City, Hertfordshire AL7 1EY (GB)**

**EP 0 403 249 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

"Slow Reacting Substance of Anaphylaxis" (SRS-A) has been shown to be a highly potent bronchoconstricting substance which is released primarily from mast cells and basophils on antigenic challenge. SRS-A has been proposed as a primary mediator in human asthma. SRS-A, in addition to its pronounced effects on lung tissue, also produces permeability changes in skin and may be involved in acute cutaneous allergic reactions. Further, SRS-A has been shown to effect depression of ventricular contraction and potentiation of the cardiovascular effects of histamine.

The discovery of the naturally occurring leukotrienes and their relationship to SRS-A has reinforced interest in SRS-A and other arachidonate metabolites SRS-A derived from mouse, rat, guinea pig and man have all been characterized as mixtures of leukotriene-$C_4$ ($LTC_4$), leukotriene-$D_4$ ($LTD_4$) and leukotriene-$E_4$ ($LTE_4$), the structural formulae of which are represented below.

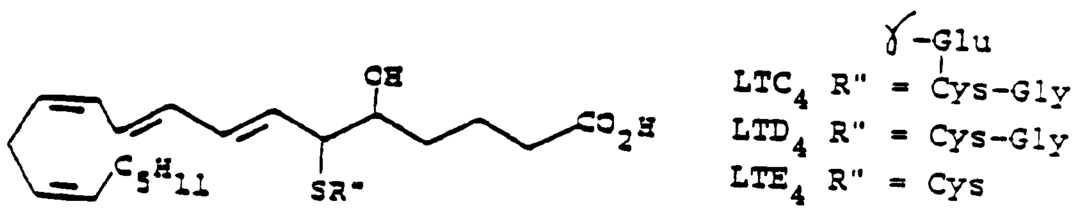

Leukotrienes are a group of eicosanoids formed from arachidonic acid metabolism via the lipoxygenase pathway. These lipid derivatives originate from $LTA_4$ and are of two types: (1) those containing a sulfido- peptide side chain ($LTC_4$, $LTD_4$, and $LTE_4$), and (2) those that are nonpeptidic ($LTB_4$). Leukotrienes comprise a group of naturally occurring substances that have the potential to contribute significantly to the pathogenesis of a variety of inflammatory and ischemic disorders. The pathophysiological role of leukotrienes has been the focus of recent intensive studies.

As summarized by Left, A.M., Biochemical Pharmacology, 35, 2, 123-127 (1986) both the peptide and non-peptide leukotrienes exert microcirculatory actions, promoting leakage of fluid across the capillary endothelial membrane in most types of vascular beds. $LTB_4$ has potent chemotactic actions and contributes to the recruitment and adherence of mobile scavenger cells to the endothelial membrane. $LTC_4$, $LTD_4$ and $LTE_4$ stimulate a variety of types of muscles. $LTC_4$ and $LTD_4$ are potent bronchoconstrictors and effective stimulators of vascular smooth muscle. This vasco-constrictor effect has been shown to occur in pulmonary, coronary, cerebral, renal, and mesenteric vasculatures.

Leukotrienes have been implicated in a number of pulmonary diseases. Leukotrienes are known to be potent bronchoconstrictors in humans. $LTC_4$ and $LTD_4$ have been shown to be potent and selective peripheral airway agonists, being more active than histamine. [See Drazen, J.M. et al., Proc. Nat'l. Acad. Sci. USA, 77, 7, 4354-4358 (1980).] $LTC_4$ and $LTD_4$ have been shown to increase the release of mucus from human airways in vitro. [See Marom, Z. et al., Am. Rev. Respir. Dis., 126, 449-451 (1982).] The leukotriene antagonists of the present invention can be useful in the treatment of allergic or non-allergic bronchial asthma or pulmonary anaphylaxis.

The presence of leukotrienes in the sputum of patients having cystic fibrosis, chronic bronchitis, and bronchiectasis at levels likely to have pathophysiological effects has been demonstrated by Zakrzewski et al. [See Zakrzewski, J.T. et al., Prostaglandins, 28, 5, 641 (1984).] Treatment of these diseases constitutes additional possible utility for leukotriene antagonists.

Leukotrienes have been identified in the nasal secretions of allergic subjects who underwent in vivo challenge with specific antigen. The release of the leukotrienes was correlated with typical allergic signs and symptoms. [See Creticos, P.S. et al., New England J. of Med., 310, 25, 1626-1629 (1984).] This suggests that allergic rhinitis is another area of utility for leukotriene antagonists.

The role of leukotrienes and the specificity and selectivity of a particular leukotriene antagonist in an animal model of the adult respiratory distress syndrome was investigated by Snapper et al. [See Snapper, J.R. et al., Abstracts of Int'l Conf. on Prostaglandins and Related Comp., Florence, Italy, p. 495 (June 1986).] Elevated concentrations of $LTD_4$ were shown in pulmonary edema fluid of patients with adult respiratory distress syndrome. [See Matthay, M. et al. J. Clin. Immunol., 4, 479-483 (1981).] Markedly elevated leukotriene levels have been shown in the edema fluid of a patient with pulmonary edema after cardiopulmonary bypass. [See Swerdlow, B.N., et al., Anesth. Analg., 65, 306-308, (1986).] LTC and LTD have also been shown to have a direct systemic arterial hypotensive effect and produce vasoconstriction and increased vasopermeability. [See Drazen et al., ibid.] This suggests leukotriene antagonists can also be useful in the areas of adult respiratory distress syndrome, pulmonary edema, and hypertension.

Leukotrienes have also been directly or indirectly implicated in a variety of non-pulmonary diseases in the ocular, dermatologic, cardiovascular, renal, trauma, inflammatory, carcinogenic and other areas.

Further evidence of leukotrienes as mediators of allergic reactions is provided by the identification of leukotrienes in tear fluids from subjects following a conjunctival provocation test and in skin blister fluids after allergen challenge in allergic skin diseases and conjunctival mucosa. [See Bisgaard, H., et al., Allergy, 40, 417-423 (1985).] Leukotriene immunoreactivity has also been shown to be present in the aqueous humor of human patients with and without uveitis. The concentrations of leukotrienes were sufficiently high that these mediators were expected to contribute in a meaningful way to tissue responses. [See Parker, J.A. et al., Arch Ophthalmol, 104, 722-724 (1986).] It has also been demonstrated that psoriatic skin has elevated levels of leukotrienes. [See Ford-Hutchinson, J. Allergy Clin. Immunol., 74, 437-440 (1984).] Local effects of intracutaneous injections of synthetic leukotrienes in human skin were demonstrated by Soter et al. [See Soter et al. J. Clin Invest Dermatol, 80, 115-119 (1983).] Cutaneous vasodilation with edema formation and a neutrophil infiltrate were induced. Leukotriene synthesis inhibitors or leukotriene antagonists can also be useful in the treatment of ocular or dermatological diseases such as allergic conjunctivitis, uveitis, allergic dermatitis or psoriasis.

Another area of utility for leukotriene antagonists is in the treatment of cardiovascular diseases. Since peptide leukotrienes are potent coronary vasoconstrictors, they are implicated in a variety of cardiac disorders including arrhythmias, conduction blocks and cardiac depression. Synthetic leukotrienes have been shown to be powerful myocardial depressants, their effects consisting of a decrease in contractile force and coronary flow. The cardiac effects of $LTC_4$ and $LTD_4$ have been shown to be antagonized by a specific leukotriene antagonist, thus suggesting usefulness of leukotriene antagonists in the areas of myocardial depression and cardiac anaphylaxis. [See Burke, J.A., et al., J. Pharmacology and Experimental Therapeutics, 221, 1, 235-241 (1982).]

$LTC_4$ and $LTD_4$ have been measured in the body fluids of rats in endotoxic shock, but are rapidly cleared from the blood into the bile. Thus leukotrienes are formed in ischemia and shock. Specific inhibitors of leukotriene biosynthesis reduce the level of leukotrienes and therefore reduce manifestations of traumatic shock, endotoxic shock, and acute myocardial ischemia. Leukotriene receptor antagonists have also been shown to reduce manifestations of endotoxic shock and to reduce extension of infarct size. Administration of peptide leukotrienes has been shown to produce significant ischemia or shock. [See Lefer, A.M., Biochemical Pharmacology, 35, 2, 123-127 (1986).] Thus further areas of utility for leukotriene antagonists can be the treatment of myocardial ischemia, acute myocardial infarction, salvage of ischemic myocardium, angina, cardiac arrhythmias, shock and atherosclerosis.

Leukotriene antagonists can also be useful in the area of renal ischemia or renal failure. Badr et al. have shown that $LTC_4$ produces significant elevation of mean arterial pressure and reductions in cardiac output and renal blood flow, and that such effects can be abolished by a specific leukotriene antagonist. [See Badr, K.R. et al., Circulation Research, 54, 492-499 (1984).] Leukotrienes have also been shown to have a role in endotoxin-induced renal failure and the effects of the leukotrienes selectively antagonized in this model of renal injury. [See Badr, K.F., et al., Kidney International, 30, 474-480 (1986).] $LTD_4$ has been shown to produce local glomerular constrictor actions which are prevented by treatment with a leukotriene antagonist. [See Badr, K.F. et al., Kidney International, 29, 1, 328 (1986).] $LTC_4$ has been demonstrated to contract rat glomerular mesangial cells in culture and thereby effect intraglomerular actions to reduce filtration surface area. [See Dunn, M.J. et al., Kidney International, 27, 1, 256 (1985).] Thus another area of utility for leukotriene antagonists can be in the treatment of glomerulonephritis.

Leukotrienes have also been indicated in the area of transplant rejection. An increase in cardiac and renal allograft survival in the presence of a leukotriene receptor antagonist was documented by Foegh et al. [See Foegh, M.L. et al. Advances in Prostaglandin, Thromboxane, and Leukotriene Research, 13, 209-217 (1985).] Rejection of rat renal allografts was shown to produce increased amounts of $LTC_4$. [See Coffman, T.M. et al., Kidney International, 29, 1, 332 (1986).]

A further area of utility for leukotriene antagonists can be in treatment of tissue trauma, burns, or fractures. A significant increase in the production of cysteinyl leukotrienes was shown after mechanical or thermal trauma sufficient to induce tissue edema and circulatory and respiratory dysfunction. [See Denzlinger, C. et al., Science, 230, 330-332 (1985).]

Leukotrienes have also been shown to have a role in acute inflammatory actions. $LTC_4$ and $LTD_4$ have potent effects on vascular caliber and permeability and $LTB_4$ increases leukocyte adhesion to the endothelium. The arteriolar constriction, plasma leakage, and leukocyte adhesion bear close resemblance to the early events in acute inflammatory reactions. [See Dahlen, S.E. et al., Proc. Natl. Acad. Sci. USA, 78, 6, 3887-3891 (1981).] Mediation of local homeostasis and inflammation by leukotrienes and other mast cell-dependent compounds was also investigated by Lewis et al. [See Lewis, R.A. et al., Nature, 293, 103-108 (1981).] Leukotriene antagonists can therefore be useful in the treatment of inflammatory diseases including rheumatoid arthritis and gout.

Cysteinyl leukotrienes have also been shown to undergo enterohepatic circulation, and thus are indicated in the area of inflammatory liver disease. [See Denzlinger, C. et al., Prostaglandins Leukotrienes and Medicine, 21, 321-322 (1986).] Leukotrienes can also be important mediators of inflammation in inflammatory bowel disease. [See Peskar, B.M. et al., Agents and Actions, 18, 381-383 (1986).] Leukotriene antagonists thus can be useful in the treatment of inflammatory liver and bowel disease.

Leukotrienes have been shown to modulate IL-1 production by human monocytes. [See Rola-Pleszczynski, M. et al., J. of Immun., 135, 6, 3958-3961 (1985).] This suggests that leukotriene antagonists may play a role in IL-1 mediated functions of monocytes in inflammation and immune reactions.

$LTA_4$ has been shown to be a factor in inducing carcinogenic tumors and is considered a link between acute immunologic defense reactions and carcinogenesis. Leukotriene antagonists can therefore possibly have utility in treatment of some types of carcinogenic tumors. [See Wischnewsky, G.G. et al. Anticancer Res. 5, 6, 639 (1985).]

Leukotrienes have been implicated in gastric cytodestruction and gastric ulcers. Damage of gastro-intestinal mucosa because of potent vasoconstriction and stasis of blood flow is correlated with increased levels of $LTC_4$. Functional antagonism of leukotriene effects may represent an alternative in treatment of mucosal injury. [See Dreyling, K.W. et al., British J. Pharmacology, 88, 236P (1986), and Peskar, B.M. et al. Prostaglandins, 31, 2, 283-293 (1986).] A leukotriene antagonist has been shown to protect against stress-induced gastric ulcer in rats. [See Ogle, C.W. et al., IRCS Med Sci., 14, 114-115 (1986).]

Other areas in which leukotriene antagonists can have utility because leukotrienes are indicated as mediators include prevention of premature labor [See Clayton, J.K. et al., Proceedings of the BPS, 573P, 17-19 Dec. 1984]; treatment of migraine headaches [See Gazzaniga, P.P. et al., Abstracts Int'l Conf. on Prostaglandins and Related Comp., 121, Florence, Italy (June 1986)]; and treatment of gallstones [See Doty, J.E. et al., Amer. J. of Surgery, 145, 54-61 (1983) and Marom, Z. et al., Amer. Rev. Respir. Dis., 126, 449-451 (1982).

By antagonizing the effects of $LTC_4$, $LTD_4$ and $LTE_4$ or other pharmacologically active mediators at the end organ, for example, airway smooth muscle, the compounds and pharmaceutical compositions of the instant invention are valuable in the treatment of diseases in subjects, including human or animals, in which leukotrienes are a key factor.

EPA 0 296 732 and EPA 0 313 697 disclose alkanoic acid compounds which are useful as leukotriene antagonists. Further compounds have now been discovered having favourable activity as leukotriene antagonists and as such are expected to be useful in the treatment of various diseases, in particular asthma.

The present invention therefore provides, in a first aspect, a compound of formula (I) or a pharmaceutically acceptable salt thereof:

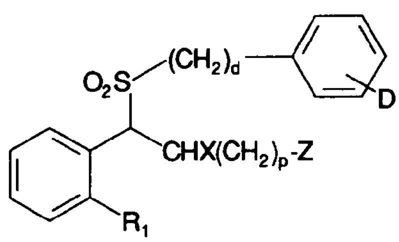

(I)

wherein $R_1$ is 8-phenyloctyl, X is OH or $C_{1-4}$alkoxy, d is 0 or 1, p is 0 or 1, Z is $COR_3$ and D is $COR_3$ where $R_3$ is OH or $C_{1-6}$alkoxy or a pharmaceutically acceptable salt thereof.

Preferably P is 0 and X is methoxy.

Preferred compound of formula (I) include [R-(R*,S*)]-3-[(4-carboxyphenylsulfonyl]-2-methoxy-3-[2-(8-phenyloctyl)phenyl]propionic acid or a pharmaceutically acceptable salt thereof.

In a further aspect the invention provides thiol compounds represented by formula (II):

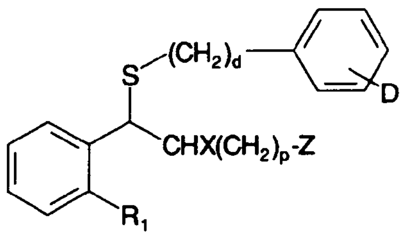

(II)

wherein X is OH or $C_{1-4}$alkoxy, $R_1$ is 8-phenyloctyl, d is 0 or 1, p is 0 or 1, Z is $COR_3$ and D is $COR_3$ where $R_3$ is OH or $C_{1-6}$alkoxy or a pharmaceutically acceptable salt thereof.

Preferred compounds of formula (II) include
2-Hydroxy-3-[(5-carboxy-2-methoxyphenylmethyl)thio]-3-[2-(8-phenyloctyl)phenyl]propionic acid;
[R-(R*,S*)]-3-[(4-carboxyphenylthio]-2-methoxy-3-[2-(8-phenyloctyl)phenyl]propionic acid;
[R-(R*,S*)]-3-[(4-carboxyphenylthio]-2-hydroxy-3-[2-(8-phenyloctyl)phenyl]propionic acid;
[R-(R*,S*)]-3-[(4-carboxy-2-methoxypheny)methylthio]-2-hydroxy-3-[2-(8-phenyloctyl)phenyl]propionic acid;
[R-(R*,S*)]-3-[(4-carboxy-2-fluorophenylmethyl)thio]-2-hydroxy-3-[2-(8-phenyloctyl)phenyl]propionic acid;
[R-(R*,S*)]-3-[(4-carboxymethylphenyl)thio]-2-hydroxy-3-[2-(8-phenyloctyl)phenyl]propionic acid,
or a pharmaceutically acceptable salt thereof.

This invention further relates to pharmaceutical compositions comprising a nontoxic effective amount of a compound of formula (I), or a pharmaceutically acceptable salt thereof, and a pharmaceutically acceptable carrier or diluent.

This invention also relates to pharmaceutical compositions for inhibiting antigen-induced respiratory anaphylaxis comprising a nontoxic effective amount of a compound of formula (I), or a pharmaceutically acceptable salt thereof, an histamine $H_1$-receptor antagonist, and a pharmaceutically acceptable carrier or diluent.

This invention also relates to a method of treating diseases in which leukotrienes are a factor in a subject in need thereof comprising administering to such subject a nontoxic effective amount of one of the above described pharmaceutical compositions.

This invention also relates to a process for preparation of the compounds of Formula (I) of known chirality comprising a) reacting an appropriate diester with a strong base to generate an intermediate thiol, and b) reacting the thiol wich an alkylating agent or Michael acceptor, i.e., $\alpha$, $\beta$ unsaturated carbonyl compound to yield a compound of Formula (I).

The compounds of the present invention, which contain one or two carboxylic acid groups, are capable of forming salts with pharmaceutically acceptable bases, according to procedures well known in the art. Such acceptable bases include organic and inorganic bases, such as ammonia, arginine, organic amines, alkaline earth and alkali metal bases. Of particular utility are the potassium, sodium, ammonium, magnesium and calcium salts.

Some of the compounds of formula (I) contain one or two asymmetric centers. This leads to the possibility of two or four stereoisomers for each such compound. The present invention includes all such stereoisomers, racemates, or mixtures thereof.

To prepare the compounds of formula (I) wherein q is 2, the appropriate thio product is conveniently oxidised with sodium periodate or meta chloroperbenzoic acid to obtain the sulphoxide product.

Alternatively the compounds of formula (I) wherein p is 0 , $R_3$ is OH and X is $C_{1-4}$alkoxy are prepared from a propenoate precursor of the following structural formula (IX)

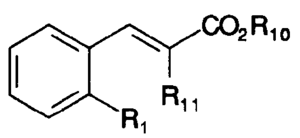

(IX)

wherein $R_1$ is as described above $R_{10}$ is a standard ester protecting group, such as t-butyl, and $R_{11}$ is $C_{1-4}$-alkoxy. A compound of formula (IX) is reacted with a mixture of alkali metal alkoxide, such as sodium methoxide, and a substituted thiol to give, after removal of the ester protective group, products of formula (I).

The propenoate precursors of formula (IX) are prepared from the corresponding aldehydes of formula (VIII)

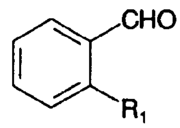

(VIII)

wherein $R_1$ is as described above by general procedures such as reaction with alkyl(triphenylphosphoranylidene)acetate or by conversion of the aldehyde to a 3-hydroxypropionate derivative, as described above, followed by an elimination reaction to form the double bond. Additionally, the propenoate precursor is obtained from a 3-methanesulphonyloxy propionate derivative by treatment with triethylamine.

The compounds of formula (I) wherein X and $R_3$ are both OH are prepared from an epoxide precursor of the following structural formula (X)

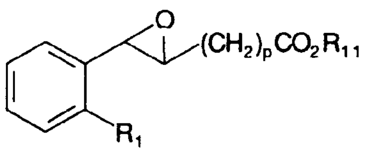

(X)

wherein $R_1$ and p are as described above, and $R_{11}$is lower alkyl, such as methyl or ethyl. A compound of formula (X) is reacted in an aprotic solvent with triethylamine and a substituted thiol selected to give, after removal of the ester protective group, a product of formula (I).

The epoxide precursors of formula (X) where p is 0 are prepared by reaction of an aldehyde of the formula (VIII) with a lower alkyl chloroacetate and an alkalimetal alkoxide such as sodium methoxide.

The compounds of formula (I) wherein X and $R_3$ are both hydroxy can also be prepared from an ester of the following structural formula (XII)

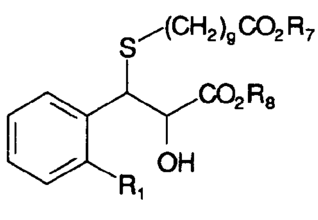

(XII)

wherein $R_7$ and $R_8$ are the same or different and are $C_{1-6}$alkyl, and g is 2. A compound of formula (XII) is reacted with sodium hydride in an inert solvent followed by reaction with a substituted benzyl bromide to yield a product of formula (I).

The aldehydes of the formula (VIII) are known or readily prepared utilizing the general procedures described as follows.

The aldehyde precursors to the compounds of the formula (I) wherein $R_1$ is, for example, an alkyl radical containing 8 to 13 carbon atoms are prepared from the appropriate 2-alkoxyphenyl-4,4-dimethyloxazoline [see Meyers et al. J. Org. Chem., 43 1372 (1978)].

The aldehyde precursors of the compounds of the formula (I) wherein $R_1$ is, for example, an alkoxy radical containing 7 to 12 carbon atoms are prepared by the O-alkylation of the appropriate 2-hydroxybenzaldehyde with the corresponding alkylating agent.

The aldehyde precursors to the compounds of the formula (I) wherein $R_1$ is a 1-alkynyl radical containing 10 to 12 carbon atoms are prepared by coupling a 2-halobenzaldehyde with the appropriate 1-alkyne in the presence of cuprous iodide and $(Ph_3)_2PdCl_2$. [See Hagihara, et al. Synthesis, 627, (1980)]. The catalytic hydrogenation of these alkynyl containing precursors under standard conditions affords the aldehyde precursors of the compounds of the formula (I) wherein $R_1$ is an alkyl or phenylalkyl radical.

The alkylthio containing aldehyde precursors of the compounds of the formula (I) are prepared by the reaction of the appropriately substituted o-haloalkylthiobenzene with magnesium and dimethylformamide.

The phenylthioalkyl containing aldehyde precursors of the compounds of the formula (I) are prepared by

the reaction of the appropriately substituted haloalkyl benzaldehyde with a thiophenol and triethylamine.

The heteroaryl mercaptan precursors necessary to prepare the compounds of formula (I) are known compounds and are conveniently prepared employing standard chemical reactions. The mercapto derivatives of these precursors are prepared according to known methods. These mercaptans are reacted as described above to yield compounds of formula (I).

Appropriate modifications of the general processes disclosed, and as further described in the Examples provided hereinbelow, furnish the various compounds defined by formula (I).

This invention further relates to a process for the preparation of the compounds of Formula (I) of known chirality comprising reacting a diester with a strong base to generate a thiol which is then reacted with an alkylating agent or Michael acceptor to yield the desired compound.

The leukotriene antagonist activity of the compounds of this invention is measured by the ability of the compounds to inhibit the leukotriene induced contraction of guinea pig tracheal tissues in vitro. The following methodology was employed:

In vitro: Guinea pig (adult male albino Hartley strain) tracheal spiral strips of approximate dimensions 2 to 3 mm cross-sectional width and 3.5 cm length were bathed in modified Krebs buffer in jacketed 10 ml tissue bath and continuously aerated with 95% $0_2$/5% $CO_2$. The tissues were connected via silk suture to force displacement transducers for recording isometric tension. The tissues were equilibrated for 1 hr., pretreated for 15 minutes with meclofenamic acid (1uM) to remove intrinsic prostaglandin responses, and then pretreated for an additional 30 minutes with either the test compound or vehicle control. A cumulative concentration-response curve for $LTD_4$ on triplicate tissues was generated by successive increases in the bath concentration of the $LTD_4$. In order to minimize intertissue variability, the contractions elicited by $LTD_4$ were standardized as a percentage of the maximum response obtained to a reference agonist, carbachol (10uM).

Calculations: The averages of the triplicate $LTD_4$ concentration-response curves both in the presence and absence of the test compound were plotted on log graph paper. The concentration of $LTD_4$ needed to elicit 30% of the contraction elicited by carbachol was measured and defined as the $EC_{30}$. The -log $K_B$ value for the test compound was determined by the following equations:

$$1. \quad \frac{EC_{30}\text{ (presence of test compound)}}{EC_{30}\text{ (presence of vehicle control)}} = \text{dose ratio} = X$$

$$2. \quad K_B = \text{concentration of test compound}/(X - 1)$$

The compounds of this invention possess biosignificant antagonist activity against leukotrienes, primarily leukotriene $D_4$. The antagonist activity of representative compounds of this invention is listed in Table I. The -log $K_B$ values were calculated from the above protocol. Where compounds were tested more than once, the -log $K_B$ values given here represent the current average data.

Table I

Leukotriene Antagonist Activity

Compounds of Formula (III) wherein $R_2$, A, C, and D are hydrogen, q is O, p is O, Z is $CO_2H$, and $R_1$ is 8-phenyloctyl

| | d | B | X | In Vitro -Log $K_B$ |
|---|---|---|---|---|
| 1) | 0 | 2-$CO_2H$ | OH | 5.5 |
| 2) | 1 | 2-$CO_2H$ | OH | 6.2 |
| 3) | 1 | 3-$CO_2H$ | OH | 6.6 |
| 4) | 0 | 3-$CO_2H$ | OH | 7.0 |
| 5) | 0 | 4-$CO_2H$ | OH | 7.6 |
| 6) | 1 | 2-$OCH_3$, 4-$CO_2H$ | OH | 7.9 |
| 7) | 1 | 4-$CO_2H$ | OH | 7.1 |
| 8) | 1 | 2-F, 4-$CO_2H$ | OH | 7.8 |
| 9) | 0 | 4-$CO_2H$ | $OCH_3$ | 7.6 |
| 10) | 0 | 4-OH | OH | 5.9 |
| 11) | 1 | 4-$CO_2H$ 2-$OCH_3$ | $OCH_3$ | 7.4 |
| 12) | 1 | 4-$CO_2H$ 2-$OCH_3$ | H | 7.5 |
| 13) | 1 | 2-$OCH_3$ 5-$CO_2H$ | OH | 6.5 |

Pharmaceutical compositions of the present invention comprise a pharmaceutical carrier or diluent and an amount of a compound of the formula (I) or a pharmaceutically acceptable salt, such as an alkali metal salt thereof, sufficient to produce the inhibition of the effects of leukotrienes, such as symptoms of asthma and other hypersensitivity diseases.

When the pharmaceutical composition is employed in the form of a solution or suspension, examples of appropriate pharmaceutical carriers or diluents include: for aqueous systems, water; for non-aqueous systems, ethanol, glycerin, propylene glycol, corn oil, cottonseed oil, peanut oil, sesame oil, liquid paraffins and mixtures thereof with water; for solid systems, lactose, kaolin and mannitol; and for aerosol systems, dichlorodifluoromethane, chlorotrifluoroethane and compressed carbon dioxide. Also, in addition to the pharmaceutical carrier or diluent, the instant compositions may include other ingredients such as stabilizers, antioxidants, preservatives, lubricants, suspending agents, viscosity modifiers and the like, provided that the additional ingredients do not have a detrimental effect on the therapeutic action of the instant compositions.

The nature of the composition and the pharmaceutical carrier or diluent will, of course, depend upon the intended route of administration, i.e. parenterally, topically or by inhalation.

In general, particularly for the prophylactic treatment of asthma, the compositions will be in a form suitable for administration by inhalation. Thus the compositions will comprise a suspension or solution of the active ingredient in water for administration by means of a conventional nebulizer. Alternatively the compositions will comprise a suspension or solution of the active ingredient in a conventional liquified propellant or compressed gas to be administered from a pressurized aerosol container. The compositions may also comprise the solid active ingredient diluted with a solid diluent for administration from a powder inhalation device. In the above compositions, the amount of carrier or diluent will vary but preferably will be the major proportion of a suspension or solution of the active ingredient. When the diluent is a solid it may be present in lesser, equal or greater amounts than the solid active ingredient.

For parenteral administration the pharmaceutical composition will be in the form of a sterile injectable solution or an aqueous or nonaqueous liquid suspension.

For topical administration the pharmaceutical composition will be in the form of a cream or ointment.

Usually a compound of formula I is administered to an animal subject in a composition comprising a nontoxic amount sufficient to produce an inhibition of the symptoms of an allergic response. When employed in this manner, the dosage of the composition is selected from the range of from 350 mg. to 700 mg. of active ingredient for each administration. For convenience, equal doses will be administered 1 to 4 times daily with the daily dosage regimen being selected from about 350 mg. to about 2800 mg.

The pharmaceutical preparations thus described are made following the conventional techniques of the pharmaceutical chemist as appropriate to the desired end product.

Included within the scope of this disclosure is the method of inhibiting the symptoms of an allergic response resulting from a mediator release which comprises administering to an animal subject a therapeutically effective amount for producing said inhibition of a compound of formula I, preferably in the form of a pharmaceutical composition. The administration may be carried out in dosage units at suitable intervals or in single doses as needed. Usually this method will be practiced when relief of allergic symptoms is specifically required. However, the method is also usefully carried out as continuous or prophylactic treatment. It is within the skill of the art to determine by routine experimentation the effective dosage to be administered from the dose range set forth above, taking into consideration such factors as the degree of severity of the allergic condition being treated, and so forth.

Compounds of this invention, alone and in combination with a histamine $H_1$-receptor antagonist, inhibit antigen-induced contraction of isolated, sensitized guinea pig trachea (a model of respiratory anaphylaxis). Exemplary of histamine $H_1$-receptor antagonists are mepyramine, chlorpheniramine, and 2-[4-(5-bromo-3-methyl-pyrid-2-yl)butylamino]-5-[(6-methyl-pyrid-3-yl)methyl]-4-pyrimidone, and other known $H_1$-receptor antagonists

Pharmaceutical compositions, as described hereinabove, of the present invention also comprise a pharmaceutical carrier or diluent and a combination of a compound of the formula (I) or a pharmaceutically acceptable salt thereof, and an histamine $H_1$-receptor antagonist in amounts sufficient to inhibit antigen-induced respiratory anaphylaxis. The above-defined dosage of a compound of formula I is conveniently employed for this purpose and the known effective dosage for the histamine $H_1$-receptor antagonist. The methods of administration described above for the single active ingredient can similarly be employed for the combination with a histamine $H_1$-receptor antagonist.

The following examples illustrate the preparation of the compounds of this invention and their incorporation into pharmaceutical compositions and as such are not to be considered as limiting the invention set forth in the claims appended hereto.

## EXAMPLE 1

### Preparation of 2-Hydroxy-3-[(5-carboxy-2-methoxyphenylmethyl)thio]-3-[2-(8-phenyloctylphenyl]propionic acid

(a) Methyl 2-hydroxy-3-[(5-carbomethoxy-2-methoxyphenylmethyl)thio]-3-[2-(8-phenyloctyl)phenyl]propionate

This compound was prepared from methyl trans-3-[2-(8-phenyloctyl)phenyl]-2,3-epoxy propionate in a manner analogous to the preparation of the compound of Example 6(b) of EPA 0 296 732.
nmr($CDCl_3$): 7.97(m,2H), 7.61(m,1H), 7.04-7.42 (m,8H), 6.87(d,1H), 4.71(t,1H), 4.519d,1H), 3.92(s,3h), 3.88(s,3H), 3.80(m, 2H), 3.64(s,3H), 3.20(d,1H), 2.60(5,2H), 2.42(m,2H), 1.12-1.83(m,12H).

(b) 2-Hydroxy-3-[(5-carboxy-2-methoxyphenylmethyl)thio]-3-[2-(8-phenyloctyl)phenyl]propionic acid

The compound of Example 1(a) was hydrolyzed in the same manner as described for the preparation of the compound of Example 4(b) of EPA 0 296 732 after tituration with a mixture of cyclohexane and hexane.
nmr($CDCl_3$): 8.10(d,1H), 7.90(d,1H), 7.52(m,1H), 6.86-7.21(m,8H), 6.89(d,1H), 4.76(d,1H), 4.48(d,1H), 3.5-3.92(m+s,5H), 2.48(t,2H), 1.98-2.38(m,2H), 0.76-1.62(m,12H).

EXAMPLE 2

Preparation of [R-(R*,S*)]-3-[(4-Carboxyphenyl)sulfonyl]-2-methoxy-3-[2-(8-phenyloctyl)phenyl]propionic acid

(a) (E)-1-(2-napthyl)-3-[2-(8-phenyloctyl)phenyl]propenone

To a 12 L 3-neck flash equipped with vigorous overhead stirring, a condenser and a cool bath (5°), under nitrogen flush, sodium metal (36.8 g., 1.16 M) was added to ethanol (3.53 L, 95%) over a period of 30 minutes. After stirring for 4 minutes, 2-phenyloctyl benzaldehyde was added. Upon completion, the reaction was recooled to 10° and the 2-acetonapthone (115.6 g., .679 M) was added in one portion. The reaction was stirred at ambient temperature for 18 h, during which time a yellow solid precipitated. The reaction was treated with icewater (350 ml) and cooled to 10°. After stirring for 1 hour, the reaction was filtered and the filter cake was washed with 50% aqueous ethanol (400 ml). The solid was air dried, pulverized and dried in vacuo (1.5 mm at room temperature) to yield 248 g. (89%) of the titled compound.
m.p.: 41-42.5]°
TLC: $R_f$ =.55 (methylene chloride; hexane 3:1,silica gel)
HPLC: RT= 17.7 min. (WATERS μBondapak®, C-18 RP;
acetonitrile: water 85:15; 1.5 ml/min.; UV detection at 230 nm)
IR(nujol mull): 1660, 1595, 1185 $cm^{-1}$
CMR[$CDCl_3$]: δ190.3, 143.3, 142.9, 142.4, 135.6, 133.5 1343.5, 132.6, 130.2, 130.15, 129.9, 129.5, 128.5, 128.3, 128.2, 127.8, 126.7, 126.6, 126.3, 125.5, 124.5, 123.3, 35.9, 33.4, 31.72, 31.4, 29.4, 29.38, 29.34, 29.23
PMR[$CDCl_3$]: δ8.55(s), 8.21(d;J=15.5 Hz), 8.11(d of d), 7.9-8.0(m), 7.8(d), 7.50-7.65(m), 7.1-7.4(m), 2.75(t; J=7.71 Hz), 2.57(5; J=7.71 Hz), 1.6(br. s), 1.29(br. s)
Elemental Analysis: Theory: C, 88.74; H, 7.67; Found: C, 88.84; H, 7.68.

(b) (2R) 1-(2-napthyl)-3-[2-(8-phenyloctyl)phenyl]trans-2,3-epoxy-propan-1-one

Sodium hydroxide (255 g, 6.37 m) was dissolved in deionized water (.65 L) which was cooled in a water bath at 14-20°C. Poly-L-leucine (215 g) was added to this solution as a solid. The chalcone (250 g, .531 M) was added as a solid followed by hexane (4 L). The heterogeneous mixture was stirred at ambient temperature for 16 hours, cooled to 10-15°C in an ice bath and ethylenediaminetetraacetic acid, disodium salt dihydrate (5 g) was added. 30% Hydrogen peroxide (1.126 L) was added dropwise over 1-2 hours in such a manner that the reaction temperature did not exceed 25°C. The peroxide was directed below the surface of the reaction by a polypropylene tube attached to a dropping funnel. The reaction was stirred at 20-24°C for 20 hours.

The reaction was treated with ehtyl acetate (.3 L) and the reaction mixture was filtered through a jacketed bench Buchner funnel (40-50°C). The solid, poly-L-leucine, was washed with ethyl acetate (.5 L), slurried in hot (40-50°C) ethyl acetate (1.5 L) for 10 minutes and collected.

The combined filtrates were placed in a separatory funnel and washed with three portions of water (550 ml each) and one portion of brine (1 L). The organic layer was dried over magnesium sulfate (300 g) for 5 hours, filtered and evaporated (30-40°C, 15 minutes) to an off-white solid.

The solid was recrystallized by dissolving in hot hexane-toluene (95-5 v/v, 1.9 L) and filtering through a jacketed bench Buchner funnel (40°C). The solution was left at ambient temperature for 1.5 hours and placed in a refrigerator (at 5°C) for 12 hours. The crystalline product was collected and washed with a small portion of the filtrate and cold hexane. The product was air dried for 3 hours and further dried in a vacuum dessicator (1 mm, 25°C) for 24 hours. This procedure yielded 200 g (82%) of the titled compound (96-97% e.e.).
m.p.: 62-63°C
TLC: Rf=0.35 ($CHCl_3$)
Rf=0.43 (methylene chloride:hexane 3:1)
HPLC: RT = 6.0 min. (Waters μBondapak® C18 RP, 3.9 mm X 30 cm; acetonitrile:water 9:1; 2 ml/min, detection at 211 nm)
RT=12.1 min. (OP(+) 4.5 mm X 25 cm; methanol; 0.8 ml/min, detection at 210 nm) enantomer RT = 18.8 min.
Elemental Analysis: theoretical, C 85.67, H 7.40; found, C 85.93, H 7.48
$[\alpha]_D$ (c=1, $CH_2Cl_2$) = + 24.6; recryx 1x, $[\alpha]_D$ = +26.4, $[\alpha]_{546}$ (c=1, $CH_2Cl_{2)}$ = +31.1.
CMR[CDCl3]: δ 193.13, 142.89, 141.47, 136.02, 133.56, 133.00, 132.49, 130.47, 129.72, 129.37, 129.06, 128.93, 128.55, 128.39, 128.22, 127.91, 127.12, 126.47, 125.56, 124.31, 123.69, 60.48, 57.67, 35.85, 32.73, 31.18, 29.39, 29.18, 29.10

(c) 2(R) 3-[2-(8-phenyloctyl)phenyl]-trans-2,3-epoxy-propionic acid, 2-napthyl ester

Methylene chloride (300 ml) was warmed to reflux and m-chloroperoxybenzoid acid (28 g, .162 M; 85% was added followed by the 2(R) 1-(2-napthyl)-2,3-(trans)-epoxy-3-[2-(8-phenyl octyl)phenyl]propanane (29g, .162 M). The reaction was stirred at reflux for 4 hours, cooled to 15 C, then m-chlorobenzoic acid was removed by filtration and the solvent was evaporated. The residue was dissolved in hot isopropanol/toluene (1L/.1L) filtered and allowed to cool at room temperature. Crystals formed within 5 minutes. The mixture was cooled in the refrigerator overnight, filtered, and air dried (25°, 1mm/Hg) to yield 25g (81%, 99.9% e.e.) of the titled product.
m.p.: 82-83°C
Specific rotation: $\alpha_D$ (c=1, $CH_2Cl_2$) = -89.48;
Elemental Analysis: theoretical, C 82.80, H 7.20; found C 82.92, H 7.09

(d) (2R) 3-(2-(8-phenyloctyl)phenyl)-2,3-(trans)-epoxy propionamide

The ester of Example 2(c) (2.03 g) was dissolved in methanol (21.1 ml) and cooled in an ice bath. Methanol saturated with ammonia (21.1 ml) was added dropwise with a temperature rise to 5°C. After 5 hours the methanol/ammonia was evaporated. The ammonia was chased by solution and evaporation from toluene. The residue was further dissolved in toluene, washed with water (1X), 10% NaOH (3X), water (2X), and brine (1X). After drying over $MgSO_4$, the solution was filtered and the solvent evaporated. The resulting solid was dissolved in hot hexane: $CH_2Cl_2$ (90:10), filtered through a hot jacketed Buchner funnel into a warmed filter flash and allowed to precipitate. After 3-1/2 hours at room temperature, the solid was collected by filtration and dried to yield 1.11 g of the titled compound.
mp. 80-82°C
Elemental Analysis: theory, C:78.59, H: 8.32, N: 3.99;
found, C: 78.35, H: 8.34, N: 3.90
$[\alpha]_D^{25}$ = -8.3 (c=1, $CH_2Cl_2$)

(e) [R-(R*,S*)]-3-[(4-carbomethoxyphenyl)thio]-2-hydroxy-3-[2-(8-phenyloctyl)phenyl]propionamide

A solution of 4.9gm (.014 mole) of the compound of Example 2(d) in 100 ml of THF at 0° was treated with 10.4 ml (.035 mole) of titanium isopropoxide, and stirred 30 minutes. This was then treated with a solution of 3.5 gm (.021 mole) of methyl-p-mercaptobenzoate in 50 ml of THF which had been treated with 100 mg of 60% NaH. The combined solution was stirred at 0° for 2 hours, poured into 400 ml of 10%. $H_2SO_4$, and extracted with $Et_2O$. The extracts were washed with $H_2O$ and aqueous $Na_2CO_3$, dried and the the solvent removed. The residue was chromatographed over silica gel, and a quantitative recovery of the product was eluted with a mixture of 10% MeOH and 90% EtOAc.
nmr ($CDCl_3$):7.93(d,2H), 7.69(m,1H), 7.46(d,2H), 6.98-7.40(m,8H), 6.30(d,1H0, 5.62(d,1H), 5.22(d,1H), 4.42(t,1H), 3.88(s,3H), 2.40-3.02(m,5H), 1.09-1.82(m,12H).

(f) Methyl[R-(R*,S*)]-3-[(4-carbomethoxyphenyl) thio]-2-hydroxy-3-[2-(8-phenyloctyl)phenyl] propionate.

A solution of 7.6 gm of the compound of Example 2(e) in a mixture of 180ml of MeOH, 15ml of conc. HCl and 10ml. of $H_2O$ was refluxed 16 hours. The MeOH was removed, and the residue extracted with $Et_2O$. The extracts were washed with $H_2O$, dried, and the solvent removed. The residue was chromatographed over a silica gel column, and 6.4 gm (89%) of the product was eluted with a mixture of 40% EtOAc and 60% hexane.
nmr($CDCl_3$): 7.94(d,2H), 7.62(m,1H), 7.40(d,2H), 6.97-7.27(m,8H), 4.95(d,1H), 4.54(t,1H), 3.82(s,3H), 3.52(s,3H), 3.13(d,1H), 2.37-2.80(m,4H), 1.03-1.87(m,12H).

(g) Methyl[R-R*,S*)]-3-[(4-carbomethoxylphenyl)thio]-2-methoxy-3-[2-(8-phenyloctyl)phenyl]propionate.

A solution of 4.08 gm (7.8 mmole) of the compound of Example 2(f) in a mixture of 80 ml of THF and 20 ml of DMF at 0° was treated first with a slurry of 343mg of 60% NcH (washed free of mineral oil with hexane) in 10 ml. of THF, and then 0.54ml (8.6 mmole) of iodomethane. After 30 minutes, the mixture was poured into 100 ml of cold 0.5N HCl, and extracted with $Et_2O$. The extracts were washed with $H_2O$ and aqueous $NaHSO_3$, dried, and the solvent evaporated. The residue was chromatographed over a silica gel column, and 3.19 gm (76%) of the product was eluted with $CHCl_3$.

nmr($CDCl_3$): 7.92(d,2H), 7.00-7.60(m,11H), 4.93(d,1H), 4.18(d,1H), 3.90(s,3H), 3.61(s,3H), 3.33(s,3H), 2.47-2.80(m,4H) 1.17-1.80(m 12H).

(h) Methyl[R-(R*,S*)]-3-[4-carbomethoxyphenyl) sulfonyl]-2-methoxy-3-[2-(8-phenyloctyl)phenyl] propionate.

A solution of 1.26 gm (2.3 mmole) of the compound of Example2 (g) in 100 ml of $CH_2Cl_2$ was treated with a solution of 1.25 gm of 80% m-chloroperbenzoic acid in 100 ml of $CH_2Cl_2$. The reaction was stirred at 23° for 1 hour, then washed twice with aqueous $Na_2CO_3$, once with aqueous $NaHSO_3$, then dried and the solvent evaporated. The residue was chromatographed over a silica gel column, and 1.22 gm (95%) of product was eluted with $CHCl_3$
nmr($CDCl_3$): 8.03(d,2H), 7.53(broad d,3H), 6.87-7.43(m,8H), 5.10(d,1H), 4.90(d,1H), 3.93(s,3H), 3.68(s,3H), 3.52(s,3H), 2.03-2.80(m,6H), 1.00-1.86(m,10H).

(i) [R-(R*,S*)]-3-[(4-Carboxyphenyl)sulfonyl]-2-methoxy-3-[2-(8-phenyloctyl)phenyl]propionic acid

A solution of 1.22 gm of the compound of Example 2(h) in a mixture of 6 ml of glacial HOAc and 3 ml of conc. HCl was refluxed 9.5 hours, cooled, and poured into 150 ml of $CHCl_3$. This solution was washed twice with $H_2O$, dried, and the solvent evaporated, and gave 0.97 gm of product.
nmr ($CDCl_3$): 8.03(d,2H), 7.53(broad d, 3H), 6.80-7.40(m,8H), 5.17(d,1H), 4.96(d,1H), 3.60(s,3H), 2.60(t,2H), 2.10-2.70(m,2H), 0.93-1.77(m,12H).

## EXAMPLE 3

Preparation of [R(R*,S*)]-3-[(4-Carboxyphenyl)thio]-2-methoxy-3-[2-(8-phenyloctyl)phenyl]propionic acid

A solution of 177 mg of the compound of Example 2(g) in 10 ml of MeOH was treated with 2ml of 2.5N NaOH and stirred 16 hours at 23°. The solution was poured into 50 ml of $H_2O$, and filtered. The filtrate was acidified and extracted with $CH_2Cl_2$. The extracts were washed with $H_2O$, dried and the solvent evaporated, and gave the product, 120 g.
nmr($CDCl_3$):7.82(d,2H), 7.58(d,2H), 7.02-7.58(m,9H), 5.10(d,1H), 4.26(d,1H), 3.20(s,3H), 2.98(m,2H), 2.45(t,2H), 1.18-1.90(m,12H).

## EXAMPLE 4

Preparation of [R-(R*,S*)]-3-[(4-Carboxyphenyl) thio]-2-hydroxy-3-[2-(8-phenyloctyl)phenyl] propionic acid

This compound was prepared from the compound of Example 2(g) in exactly the same manner as used in Example 3.
nmr:($CDCl_3/Me_2CO$):8.00(d,2H), 7.70(m,1H), 7.49(d,2H), 7.00-7.45(m,8H), 5.12(d,1H), 4.67(d,1H), 2.40-2.90(m,4H), 1.10-1.76(n,12H).

## EXAMPLE 5

Preparation of [R-(R*, S*)]-3-[(4-carboxy-2-methoxyphenylmethyl)thio]-2-hydroxy-3-[2-(8-phenyloctyl)phenyl]propionic acid

(a) [R-(R*,S*)]-3-[(4-carbomethoxy-2-methoxyphenylmethylthio]-2-hydroxy-3-[2-(8-phenyloctyl)phenyl]propionamide

A solution of 2.76 gm (13 mmole) of 4-carbomethoxy-2-methoxybenzylmercaptan in 70 ml of THF at 0° was treated with 520mg (13 mmole) 60% NaH. A solution of 3.51 gm (10 mmole) of the compound of Example 19(d) in 100 ml of $CH_2Cl_2$ at 0° was treated with 7.43 ml (25 mmole) of titanium isopropoxide. The two solutions were combined and stirred at 0° for 2 hours, at 20° for 16 hours, and quenched with 100 ml of 10% $H_2SO_4$. The mixture was extracted with $CH_2Cl_2$. The extracts were washed with $H_2O$, dried, and the solvent evaporated. The residue was chromatographed over a silica column, and 3.4 gm of product was eluted with a mixture of 5% MeOH and 95% EtOAc.

(b) Methyl[R-(R*,S*)]-3-[(4-carbomethoxy-2-methoxy phenylmethyl)thio]-2-hydroxy-3[2-(8-phenyloctyl) phenyl]propionate

This compound is prepared from the compound of Example 5(a) in a manner exactly analagous to the preparation of the compound of Example 2(f).
nmr($CDCl_3$): 6.92-7.78(m,12H), 4.70(t,1H), 4.50(d,1H), 3.60-3.93(d of d, 2H), 3.90(s,3H), 3.87(s,3H), 3.60(s,3H), 3.30(d,1H), 2.58(t,2H), 2.20-2.46(m,2H), .98-1.72(m,12H).

(c) [R-(R*,S*)]-3-[(4-carboxy-2-methoxyphenylmethyl)thio]-2-hydroxy-3-[2-(8-phenyloctyl) phenyl]propionic acid

This compound was prepared from the compound of Example 5(b) in a manner exactly analagous to the preparation of the compound of Example 3.
nmr($CDCl_3/Me_2Co$): 6.93-7.78(m,12H), 4.76(d,1H), 4.53(d,1H), 3.92(d,1H), 3.82(s,3H), 3.68(d,1H), 2.24-2.69(m,4H), 1.03-1.80(m,12H).

EXAMPLE 6

Preparation of [R-(R*,S*)]-3-[(4-carboxy-2-fluorophenylmethyl)thio]-2-hydroxy-3-[2-(8-phenyloctyl)phenyl]propionic acid

(a) [R-(R*,S*)]-3-[(4-carbomethoxy-2-fluorophenylmethyl)thio]-2-hydroxy-3-[2-(8-phenyloctyl)phenyl]propionamide

This compound was prepared from the compound of Example 2(c) in a manner exactly analagous to that of Example 5.

(b) Methyl [R-(R*,S*)]-3-[(4-carbomethoxy-2-fluorophenylmethyl) thio]-2-hydroxy-3-[2-(8-phenyloctyl)phenyl]propionate

This compound was prepared from the compound of Example 6(a) in a manner exactly analagous to the preparation of Example 2(f).
nmr($CDCl_3$): 6.97-7.90(m,12H), 4.42-4.72(m,2H), 3.48-3.98(m,8H), 3.06(d,1H), 2.28-2.72(m,4H), 1.00-1.749m,12H).

(c) [R-(R*,S*)]-3-[(4-carboxy-2-fluorophenylmethyl)thio]-2-hydroxy-3-[2-(8-phenyloctyl)phenyl]propionic acid

This compound was prepared from the compound of Example 6(b) in a manner exactly analagous to the preparation of the compound of Example 3.
nmr($CDCl_3$): 6.98-7.90(m,12H), 4.68(d,1H), 4.55(d,1H), 3.60-3.92(m,2H), 2.20-2.70(m,4H), 1.02-1.76 (m,12H).

EXAMPLE 7

Preparation of [R-(R*,S*)]-3-[(3-carboxymethylphenyl)thio]-2-hydroxy-3-[2-(8-phenyloctyl)phenyl]propionic acid

(a) [R-(R*,S*)]-3-[(3-carbomethoxymethylphenyl)thio]-2-hydroxy-3-[2-(8-phenyloctyl)phenyl]propionamide.

This compound was prepared from the compound of Example 2(d) and methyl-m-mercaptophenylacetate in a manner exactly analagous to the preparation of the compound of Example 5(a).
nmr($CDCl_3$): 7.58-7.82(m,1H), 6.96-7.52(m,12H), 6.39(broad d, 1H), 5.62(broad d,1H), 5.05(d,1H), 4.37(t,1H), 4.08(d,1H), 3.70(s,3H), 3.58(s,2H), 2.50-3.02(m,4H), 1.18-1.82(m,12H).

(b) Methyl [R-(R*,S*)]-3-[(3-carbomethoxymethyl phenyl)thio]-2-hydroxy-3-[2-(8-phenyloctyl) phenyl]propionate

This compound was prepared from the compound of Example 7(a) in a manner exactly analagous to the preparation of the compound of Example 2(f).

(c) [R-(R*,S*)]-3-[(3-carboxymethylphenyl)thio]-2-hydroxy-3-[2-(8-phenyloctyl)phenyl]propionic acid

This compound was prepared from the compound of Example 5(b) in a manner exactly analagous to the preparation of the compound of Example 3.
nmr($CDCl_3$): 7.50-7.78(m,1H), 6.72-7.48(m,12H), 4.90(d,1H), 4.50(d,1H), 3.52(s,2H), 2.32-2.86(m,4H), 0.94-1.72(m,12H)

EXAMPLE 8

As a specific embodiment of a composition of this invention, 1 to 10 mg/ml of an active ingredient, such as the compound of Example 1 is dissolved in isotnoic saline solution and aerosolized from a nebulizer operating at an air flow adjusted to deliver the desired aerosolized weight of drug.

EXAMPLE 9

As an additional embodiment of a composition of this invention 100 mg of an active ingredient, such as the compound of Example 4, is combined with 4 mg of chlorpheniramine maleate with a suitable carrier or excipient.

## Claims

**Claims for the following Contracting States : AT, BE, CH, DE, DK, FR, GB, IT, LI, LU, NL, SE**

1. A compound represented by the following structural formula (I):

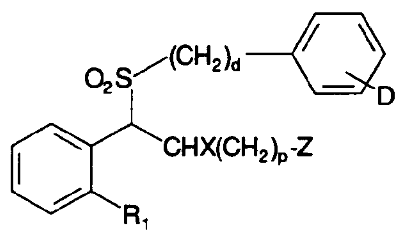

(I)

wherein $R_1$ is 8-phenyloctyl, X is OH or $C_{1-4}$alkoxy, d is 0 or 1, p is 0 or 1, Z is $COR_3$ and D is $COR_3$ where $R_3$ is OH or $C_{1-6}$alkoxy or a pharmaceutically acceptable salt thereof.

2. A compound according to claim 1 in which P is 0 and X is methoxy.

3. A compound according to claim 2 which is [R-(R*,S*)]-3-[(4-carboxyphenylsulfonyl]-2-methoxy-3-[2-(8-phenyloctyl)phenyl]propionic acid or a pharmaceutically acceptable salt thereof.

4. A compound represented by formula (II):

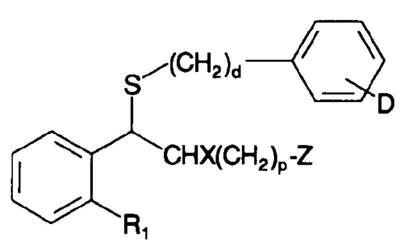

(II)

wherein X is OH or $C_{1-4}$alkoxy, $R_1$ is 8-phenyloctyl, d is 0 or 1, p is 0 or 1, Z is $COR_3$ and D is $COR_3$ where $R_3$ is OH or $C_{1-6}$alkoxy or a pharmaceutically acceptable salt thereof.

5. A compound according to claim 4 which is:
2-Hydroxy-3-[(5-carboxy-2-methoxyphenylmethyl)thio]-3-[2-(8-phenyloctyl)phenyl]propionic acid;
[R-(R*,S*)]-3-[(4-carboxyphenylthio]-2-methoxy-3-[2-(8-phenyloctyl)phenyl]propionic acid;
[R-(R*,S*)]-3-[(4-carboxyphenylthio]-2-hydroxy-3-[2-(8-phenyloctyl)phenyl]propionic acid;
[R-(R*,S*)]-3-[(4-carboxy-2-methoxypheny)methylthio]-2-hydroxy-3-[2-(8-phenyloctyl)phenyl]propionic acid;
[R-(R*,S*)]-3-[(4-carboxy-2-fluorophenylmethyl)thio]-2-hydroxy-3-[2-(8-phenyloctyl)phenyl]propionic acid;
[R-(R*,S*)]-3-[(4-carboxymethylphenyl)thio]-2-hydroxy-3-[2-(8-phenyloctyl)phenyl]propionic acid,
or a pharmaceutically acceptable salt thereof.

6. A compound according to any one of claims 1 to 5 for use as a medicament.

7. A pharmaceutical composition comprising a pharmaceutical carrier or diluent and a nontoxic effective amount of a compound according to any one of claims 1 to 5.

8. The use of a compound according to any one of claims 1 to 5 or a pharmaceutically acceptable salt thereof in the manufacture of a medicament for the treatment of asthma.

9. A process for preparing a compound of formula (I) or a pharmaceutically acceptable salt thereof as defined in claim I, which process comprises oxidising the sulfur atom of a compound of formula (XVIII):

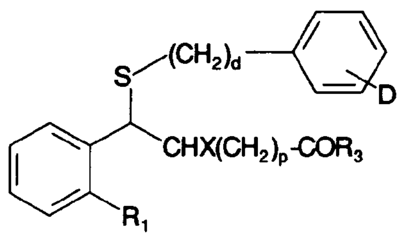

(XVIII)

where X is OH or $C_{1-4}$alkoxy and $R_1$, $R_3$, d and p are as defined in claim 1 and optionally thereafter forming a pharmaceutically acceptable salt.

10. A process for preparing a compound of formula (II) or a pharmaceutically acceptable salt thereof as defined in claim 4, which process comprises:
A) reacting a strong base with a compound of formula (XIV):

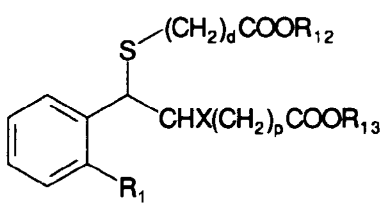

(XIV)

wherein d is 1, X is OH, $R_1$ and p are as defined in claim 4 and $R_{12}$ and $R_{13}$ are independently $C_{1-4}$alkyl to yield a thiol of formula (XV):

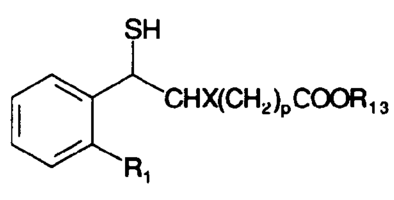

(XV)

wherein X, $R_1$ and p are as defined above in formula (XIV), and reacting the thiol of formula (XV) with an alkylating agent to yield a compound of formula (I); or

B) reacting an epoxide of formula (XVI)

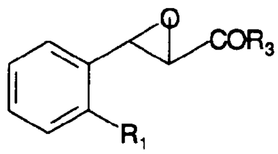

(XVI)

with a group HS-R in the presence of a titanium tetra-alkoxide to obtain a compound of formula (I) where X is OH: or

C) etherifying a compound of formula (XVII):

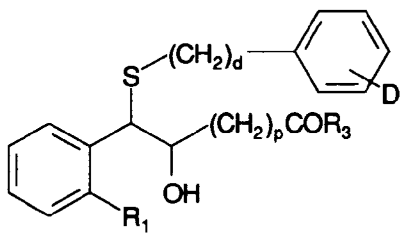

(XVII)

wherein d, X, $R_1$ and p are as defined above with an alkanol.

**11.** A process according to claim 10B wherein the mercapto-containing group is

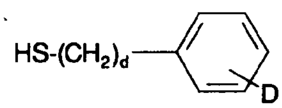

where d is 0 or 1 and D is as defined above and the titanium tetra-alkoxide is titanium isopropoxide.

**Claims for the following Contracting States : ES, GR**

**1.** A process for preparing a compound of formula (I) or a pharmaceutically acceptable salt thereof:

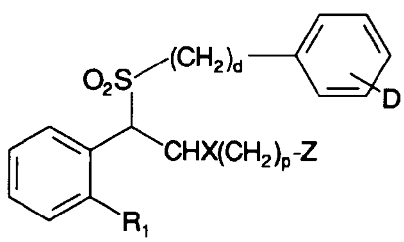

(I)

wherein $R_1$ is 8-phenyloctyl, X is OH or $C_{1-4}$alkoxy, d is 0 or 1, p is 0 or 1, Z is $COR_3$ and D is $COR_3$ where $R_3$ is OH or $C_{1-6}$alkoxy which process comprises oxidising the sulfur atom of a compound of formula (XVIII):

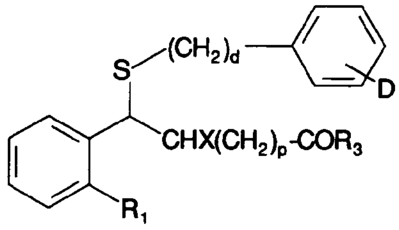

(XVIII)

where X is OH or $C_{1-4}$alkoxy and $R_1$, $R_3$, d and p are as defined in formula (I), and optionally thereafter forming a pharmaceutically acceptable salt.

2. A process according to claim 1 in which P is 0 and X is methoxy.

3. A process according to claim 2 in which the compound prepared is [R-(R*,S*)]-3-[(4-carboxyphenylsulfonyl]-2-methoxy-3-[2-(8-phenyloctyl)phenyl]-propionic acid or a pharmaceutically acceptable salt thereof.

4. A process for preparing a compound of formula (II) or a pharmaceutically acceptable salt thereof:

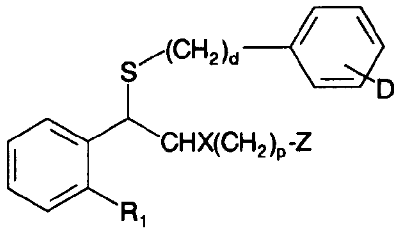

(II)

wherein X is OH or $C_{1-4}$alkoxy, $R_1$ is 8-phenyloctyl, d is 0 or 1, p is 0 or 1, Z is $COR_3$ and D is $COR_3$ where $R_3$ is OH or $C_{1-6}$alkoxy which process comprises:

A) reacting a strong base with a compound of formula (XIV):

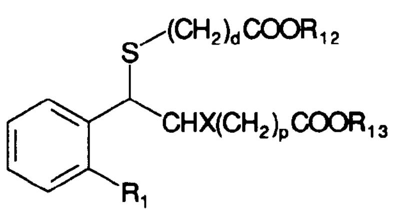

(XIV)

wherein d is 1, X is OH, $R_1$ and p are as defined in claim 1 and $R_{12}$ and $R_{13}$ are independently $C_{1-4}$alkyl to yield a thiol of formula (XV):

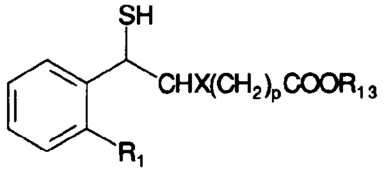

(XV)

wherein X, $R_1$ and p are as defined above in formula (XIV), and reacting the thiol of formula (XV) with an alkylating agent to yield a compound of formula (I); or

B) reacting an epoxide of formula (XVI)

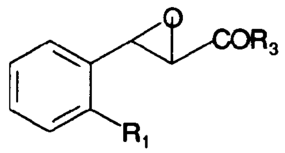

(XVI)

with a group HS-R in the presence of a titanium tetra-alkoxide to obtain a compound of formula (I) where X is OH: or

C) etherifying a compound of formula (XVII):

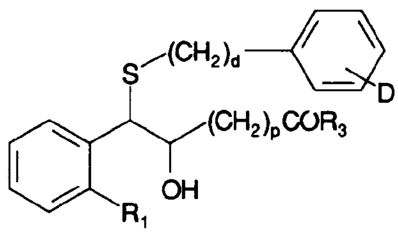

(XVII)

wherein d, X, $R_1$ and p are as defined above with an alkanol.

5. A process according to claim 4B in which the HS-R group is

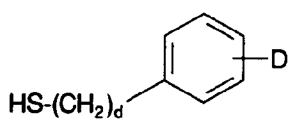

where d is 1 and D is as defined above and the titanium tetra-alkoxide is titanium isopropoxide.

6. A process according to claim 4 or 5 in which the compound prepared is:
2-Hydroxy-3-[(5-carboxy-2-methoxyphenylmethyl)thio]-3-[2-(8-phenyloctyl)phenyl]propionic acid;
[R-(R*,S*)]-3-[(4-carboxyphenylthio]-2-methoxy-3-[2-(8-phenyloctyl)phenyl]propionic acid;
[R-(R*,S*)]-3-[(4-carboxyphenylthio]-2-hydroxy-3-[2-(8-phenyloctyl)phenyl]propionic acid;
[R-(R*,S*)]-3-[(4-carboxy-2-methoxypheny)methylthio]-2-hydroxy-3-[2-(8-phenyloctyl)phenyl]propionic acid;
[R-(R*,S*)]-3-[(4-carboxy-2-fluorophenylmethyl)thio]-2-hydroxy-3-[2-(8-phenyloctyl)phenyl]propionic acid;
[R-(R*,S*)]-3-[(4-carboxymethylphenyl)thio]-2-hydroxy-3-[2-(8-phenyloctyl)phenyl]propionic acid,
or a pharmaceutically acceptable salt thereof.

7. A process for preparing a pharmaceutical composition which comprises bringing into association a compound of formula (I) or a pharmaceutically acceptable salt thereof as defined in claim 1 and a pharmaceutically acceptable carrier.

8. The use of a compound of formula (I) as defined in claim 1 in the manufacture of a medicament for the treatment of asthma.

9. A process for preparing a pharmaceutical composition which comprises bringing into association a compound of formula (II) or a pharmaceutically acceptable salt thereof as defined in claim 4 and a pharmaceutically acceptable carrier.

10. The use of a compound of formula (II) as defined in claim 4 in the manufacture of a medicament for the

treatment of asthma.

## Patentansprüche

### Patentansprüche für folgende Vertragsstaaten : AT, BE, CH, DE, DK, FR, GB, IT, LI, LU, NL, SE

1. Verbindung der folgenden Strukturformel (I),

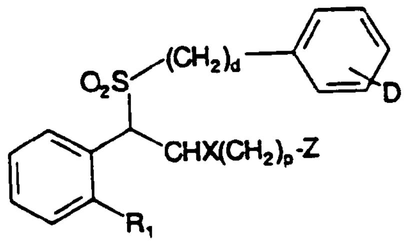

(I)

in der $R_1$ eine 8-Phenyloctylgruppe bedeutet, X eine OH-Gruppe oder einen $C_{1-4}$-Alkoxyrest darstellt, d 0 oder 1 ist, p 0 oder 1 ist, Z einen Rest der Formel $COR_3$ darstellt und D einen Rest der Formel $COR_3$ bedeutet, wobei $R_3$ eine OH-Gruppe oder einen $C_{1-6}$-Alkoxyrest bedeutet, oder ein pharmazeutisch verträgliches Salz davon.

2. Verbindung nach Anspruch 1, wobei p 0 ist und X eine Methoxygruppe bedeutet.

3. Verbindung nach Anspruch 2, die [R-(R*,S*)]-3-[(4-Carboxyphenyl)sulfonyl]-2-methoxy-3-[2-(8-phenyloctyl)phenyl]propionsäure oder ein pharmazeutisch verträgliches Salz davon ist.

4. Verbindung der Formel (II),

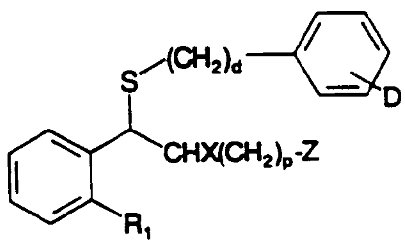

(II)

in der X eine OH-Gruppe oder einen $C_{1-4}$-Alkoxyrest darstellt, $R_1$ eine 8-Phenyloctylgruppe bedeutet, d 0 oder 1 ist, p 0 oder 1 ist, Z einen Rest der Formel $COR_3$ darstellt und D einen Rest der Formel $COR_3$ bedeutet, wobei $R_3$ eine OH-Gruppe oder einen $C_{1-6}$-Alkoxyrest bedeutet, oder ein pharmazeutisch verträgliches Salz davon.

5. Verbindung nach Anspruch 4, die
2-Hydroxy-3-[(5-carboxy-2-methoxyphenylmethyl)thio]-3-[2-(8-phenyloctyl)phenyl]propionsäure,
[R-(R*,S*)]-3-[(4-Carboxyphenyl)thio]-2-methoxy-3-[2-(8-phenyloctyl)phenyl]propionsäure,
[R-(R*,S*)]-3-[(4-carboxyphenyl)thio]-2-hydroxy-3-[2-(8-phenyloctyl)phenyl]propionsäure,
[R-(R*,S*)]-3-[(4-carboxy-2-methoxyphenyl)methylthio]-2-hydroxy-3-[2-(8-phenyloctyl)phenyl]propionsäure,
[R-(R*,S*)]-3-[(4-Carboxy-2-fluorphenylmethyl)thio]-2-hydroxy-3-[2-(8-phenyloctyl)phenyl]propionsäure,
[R-(R*,S*)]-3-[(4-Carboxymethylphenyl)thio]-2-hydroxy-3-[2-(8-phenyloctyl)phenyl]propionsäure,
oder ein pharmazeutisch verträgliches Salz davon ist.

6. Verbindung nach einem der Ansprüche 1 bis 5 zur Verwendung als Medikament.

7. Arzneimittel, das einen pharmazeutischen Träger oder ein pharmazeutisches Verdünnungsmittel und eine nichttoxische wirksame Menge einer Verbindung nach einem der Ansprüche 1 bis 5 umfaßt.

8. Verwendung einer Verbindung nach einem der Ansprüche 1 bis 5 oder eines pharmazeutisch verträglichen Salzes davon zur Herstellung eines Medikaments für die Behandlung von Asthma.

9. Verfahren zur Herstellung einer Verbindung der Formel (I) oder eines pharmazeutisch verträglichen Salzes davon nach Anspruch 1, wobei das Verfahren die Oxidation des Schwefelatoms einer Verbindung der Formel (XVIII),

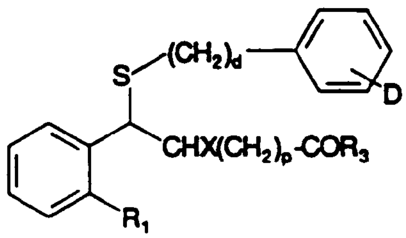

(XVIII)

in der X eine OH-Gruppe oder einen $C_{1-4}$-Alkoxyrest bedeutet und $R_1$, $R_3$, d und p wie in Anspruch 1 definiert sind, und gegebenenfalls danach die Erzeugung eines pharmazeutisch verträglichen Salzes umfaßt.

10. Verfahren zur Herstellung eine Verbindung der Formel (II) oder eines pharmazeutisch verträglichen Salzes davon nach Anspruch 4, wobei das Verfahren
A) die Umsetzung einer starken Base mit einer Verbindung der Formel (XIV),

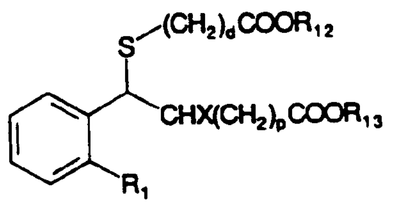

(XIV)

in der d 1 ist, X eine OH-Gruppe darstellt, $R_1$ und p wie in Anspruch 4 definiert sind und $R_{12}$ und $R_{13}$ unabhängig $C_{1-4}$-Alkylreste bedeuten, zum Erhalt eines Thiols der Formel (XV),

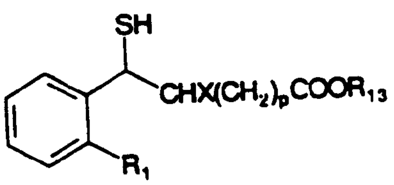

(XV)

in der X, $R_1$ und p wie vorstehend in Formel (XIV) definiert sind, und die Umsetzung des Thiols der Formel (XV) mit einem Alkylierungsmittel zum Erhalt einer Verbindung der Formel (I); oder
B) die Umsetzung eines Epoxids der Formel (XVI)

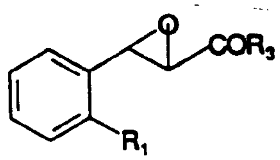

(XVI)

mit einem Rest der Formel HS-R in Gegenwart eines Titantetraalkoxids zum Erhalt einer Verbindung der Formel (I), wobei X eine OH-Gruppe bedeutet; oder
C) die Veretherung einer Verbindung der Formel (XVII),

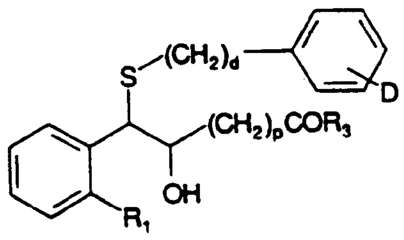

(XVII)

in der d, X, $R_1$ und p wie vorstehend definiert sind, mit einem Alkanol umfaßt.

**11.** Verfahren nach Anspruch 10B, wobei die Mercapto-enthaltende Gruppe

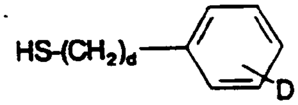

bedeutet, in der d 0 oder 1 ist, D wie vorstehend definiert ist und das Titantetraalkoxid Titanisopropoxid ist.

**Patentansprüche für folgende Vertragsstaaten : ES, GR**

**1.** Verfahren zur Herstellung einer Verbindung der Formel (I) oder eines pharmazeutisch verträglichen Salzes davon,

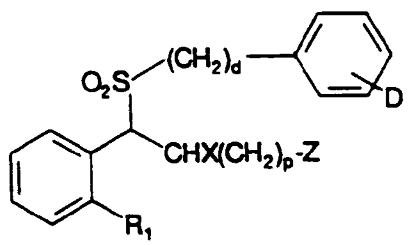

(I)

wobei $R_1$ eine 8-Phenyloctylgruppe bedeutet, X eine OH-Gruppe oder einen $C_{1-4}$-Alkoxyrest darstellt, d 0 oder 1 ist, p 0 oder 1 ist, Z einen Rest der Formel $COR_3$ darstellt und D einen Rest der Formel $COR_3$ bedeutet, in der $R_3$ eine OH-Gruppe oder einen $C_{1-6}$-Alkoxyrest darstellt, wobei das Verfahren die Oxidation des Schwefelatoms einer Verbindung der Formel (XVIII),

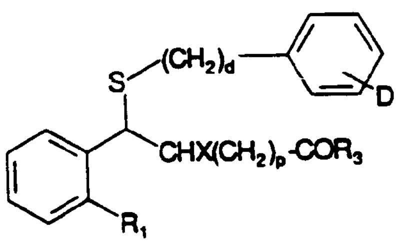

(XVIII)

in der X eine OH-Gruppe oder einen $C_{1-4}$-Alkoxyrest bedeutet und $R_1$, $R_3$, d und p wie in Formel (I) definiert sind, und gegebenenfalls danach die Erzeugung eines pharmazeutisch verträglichen Salzes umfaßt.

**2.** Verfahren nach Anspruch 1, wobei p 0 ist und X eine Methoxygruppe bedeutet.

**3.** Verfahren nach Anspruch 2, wobei die hergestellte Verbindung [R-(R*,S*)]-3-[(4-Carboxyphenyl)sulfonyl]-2-methoxy-3-[2-(8-phenyloctyl)phenyl]propionsäure oder ein pharmazeutisch verträgliches Salz davon ist.

4. Verfahren zur Herstellung einer Verbindung der Formel (II) oder eines pharmazeutisch verträglichen Salzes davon,

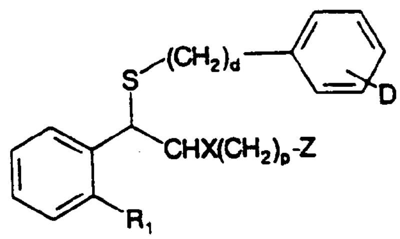

(II)

in der X eine OH-Gruppe oder einen $C_{1-4}$-Alkoxyrest darstellt, $R_1$ eine 8-Phenyloctylgruppe bedeutet, d 0 oder 1 ist, p 0 oder 1 ist, Z einen Rest der Formel $COR_3$ darstellt und D einen Rest der Formel $COR_3$ bedeutet, in der $R_3$ eine OH-Gruppe oder einen $C_{1-6}$-Alkoxyrest darstellt, wobei das Verfahren

A) die Umsetzung einer starken Base mit einer Verbindung der Formel (XIV),

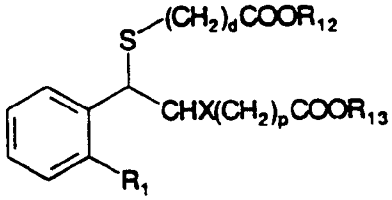

(XIV)

in der d 1 ist, X eine OH-Gruppe darstellt, $R_1$ und p wie in Anspruch 1 definiert sind und $R_{12}$ und $R_{13}$ unabhängig $C_{1-4}$-Alkylreste bedeuten, zum Erhalt eines Thiols der Formel (XV),

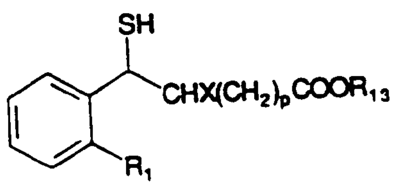

(XV)

in der X, $R_1$ und p wie vorstehend in Formel (XIV) definiert sind, und die Umsetzung des Thiols der Formel (XV) mit einem Alkylierungsmittel zum Erhalt einer Verbindung der Formel (I); oder

B) die Umsetzung eines Epoxids der Formel (XVI)

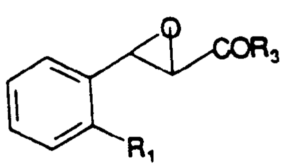

(XVI)

mit einem Rest der Formel HS-R in Gegenwart eines Titantetraalkoxids zum Erhalt einer Verbindung der Formel (I), wobei X eine OH-Gruppe bedeutet; oder

C) die Veretherung einer Verbindung der Formel (XVII),

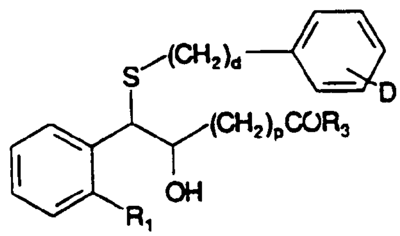

(XVII)

in der d, X, $R_1$ und p wie vorstehend definiert sind, mit einem Alkanol umfaßt.

5. Verfahren nach Anspruch 4B, wobei der Rest der Formel HS-R

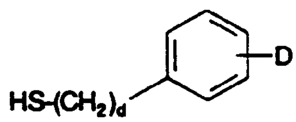

bedeutet, in der d 1 ist, D wie vorstehend definiert ist und das Titantetraalkoxid Titanisopropoxid ist.

6. Verfahren nach Anspruch 4 oder 5, wobei die hergestellte Verbindung
2-Hydroxy-3-[(5-carboxy-2-methoxyphenylmethyl)thio]-3-[2-(8-phenyloctyl)phenyl]propionsäure,
[R-(R*,S*)]-3-[(4-Carboxyphenyl)thio]-2-methoxy-3-[2-(8-phenyloctyl)phenyl]propionsäure,
[R-(R*,S*)]-3-[(4-Carboxyphenyl)thio]-2-hydroxy-3-[2-(8-phenyloctyl)phenyl]propionsäure,
[R-(R*,S*)]-3-[(4-Carboxy-2-methoxyphenyl)methylthio]-2-hydroxy-3-(2-(8-phenyloctyl)phenyl]propionsäure,
[R-(R*,S*)]-3-[(4-Carboxy-2-fluorphenylmethyl)thio]-2-hydroxy-3-[2-(8-phenyloctyl)phenyl]propionsäure,
[R-(R*,S*)]-3-[(4-Carboxymethylphenyl)thio]-2-hydroxy-3-[2-(8-phenyloctyl)phenyl]propionsäure,
oder ein pharmazeutisch verträgliches Salz davon ist.

7. Verfahren zur Herstellung eines Arzneimittels, das das Zusammenbringen einer Verbindung der Formel (I) oder eines pharmazeutisch verträglichen Salzes davon nach Anspruch 1 und eines pharmazeutisch verträglichen Trägers umfaßt.

8. Verwendung einer Verbindung der Formel (I) nach Anspruch 1 zur Herstellung eines Medikaments für die Behandlung von Asthma.

9. Verfahren zur Herstellung eines Arzneimittels, das das Zusammenbringen einer Verbindung der Formel (II) oder eines pharmazeutisch verträglichen Salzes davon nach Anspruch 4 und eines pharmazeutisch verträglichen Trägers umfaßt.

10. Verwendung einer Verbindung der Formel (II) nach Anspruch 4 zur Herstellung eines Medikaments für die Behandlung von Asthma.

## Revendications

### Revendications pour les Etats contractants suivants : AT, BE, CH, DE, DK, FR, GB, IT, LI, LU, NL, SE

1. Composé représenté par la formule structurale suivante (I) :

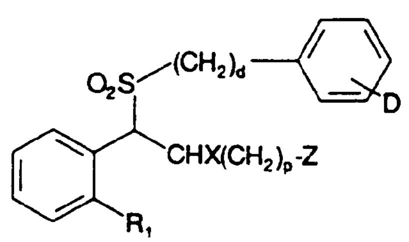

(I)

dans laquelle $R_1$ est un groupe 8-phényloctyle, X est un groupe OH ou alcoxy en $C_{1-4}$, d est 0 ou 1, p est 0 ou 1, Z est $COR_3$ et D est $COR_3$ dans lesquels $R_3$ est un groupe OH ou alcoxy en $C_{1-6}$, ou sel de celui-ci acceptable du point de vue pharmaceutique.

**2.** Composé suivant la revendication 1, dans lequel p est 0 et X est un groupe méthoxy.

**3.** Composé suivant la revendication 2, qui est l'acide [R-(R*,S*)]-3-[(4-carboxyphényl)sulfonyl]-2-méthoxy-3-[2-(8-phényloctyl)-phényl]-propionique ou un sel de celui-ci acceptable du point de vue pharmaceutique.

**4.** Composé représenté par la formule (II) :

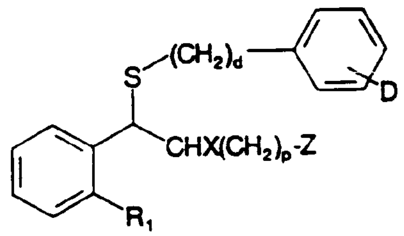

(II)

dans laquelle $R_1$ est un groupe 8-phényloctyle, X est un groupe OH ou alcoxy en $C_{1-4}$, d est 0 ou 1, p est 0 ou 1, Z est $COR_3$ et D est $COR_3$ dans lesquels $R_3$ est un groupe OH ou alcoxy en $C_{1-6}$, ou sel de celui-ci acceptable du point de vue pharmaceutique.

**5.** Composé suivant la revendication 4, qui est:

l'acide 2-hydroxy-3-[(5-carboxy-2-méthoxyphénylméthyl)-thio]-3-[2-(8-phényloctyl)-phényl]-propionique;

l'acide [R-(R*,S*)]-3-[(4-carboxyphényl)-thio]-2-méthoxy-3-[2-(8-phényloctyl)-phényl]-propionique;

l'acide [R-(R*,S*)]-3-[(4-carboxyphényl)-thio]-2-hydroxy-3-[2-(8-phényloctyl)-phényl]-propionique;

l'acide [R-(R*,S*)]-3-[(4-carboxy-2-méthoxyphénylméthyl)-thio]-2-hydroxy-3-[2-(8-phényloctyl)-phényl]-propionique;

l'acide [R-(R*,S*)]-3-[(4-carboxy-2-fluorophénylméthyl)-thio]-2-hydroxy-3-[2-(8-phényloctyl)-phényl]-propionique;

l'acide [R-(R*,S*)]-3-[(4-carboxyméthylphényl)-thio]-2-hydroxy-3-[2-(8-phényloctyl)-phényl]-propionique;

ou un sel de ceux-ci acceptable du point de vue pharmaceutique.

**6.** Composé suivant l'une quelconque des revendications 1 à 5, utilisable comme médicament.

**7.** Composition pharmaceutique comprenant un support ou un diluant pharmaceutique et une quantité efficace non toxique d'un composé suivant l'une quelconque des revendications 1 à 5.

**8.** Utilisation d'un composé suivant l'une quelconque des revendications 1 à 5 ou d'un sel de celui-ci acceptable du point de vue pharmaceutique, dans la fabrication d'un médicament pour le traitement de l'asthme.

**9.** Procédé pour la préparation d'un composé de formule (I) ou d'un sel de celui-ci acceptable du point de vue pharmaceutique suivant la revendication 1, qui comprend l'oxydation de l'atome de soufre d'un composé de formule (XVIII) :

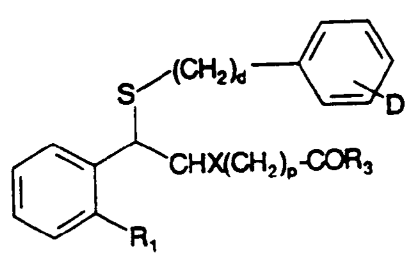

(XVIII)

dans laquelle X est un groupe OH ou alcoxy en $C_{1-4}$, et $R_1$, $R_3$, d et p sont tels que définis dans la revendication 1, et la formation éventuelle d'un sel acceptable du point de vue pharmaceutique.

**10.** Procédé pour la préparation d'un composé de formule (II) ou d'un sel de celui-ci acceptable du point de vue pharmaceutique suivant la revendication 4, qui comprend :

A) la réaction d'une base forte avec un composé de formule (XIV) :

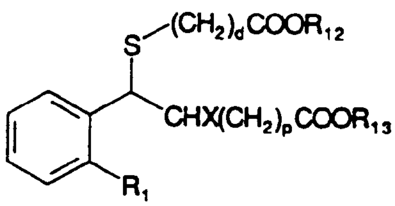

(XIV)

dans laquelle d est 1, X est un groupe OH, $R_1$ et p sont tels que définis dans la revendication 4 et $R_{12}$ et $R_{13}$ sont indépendamment un groupe alkyle en $C_{1-4}$ pour fournir un thiol de formule (XV) :

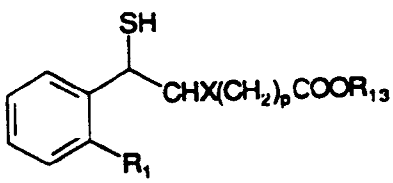

(XV)

dans laquelle X, $R_1$ et p sont tels que définis plus haut dans la formule (XIV) et la réaction du thiol de formule (XV) avec un agent alkylant pour fournir un composé de formule (I); ou

B) la réaction d'un époxyde de formule (XVI) :

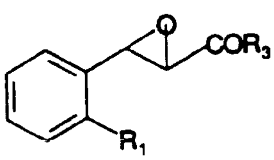

(XVI)

avec un composé HS-R en présence de tétraalcoolate de titane pour obtenir un composé de formule (I) dans laquelle X est un groupe OH; ou

C) l'éthérification d'un composé de formule (XVII) :

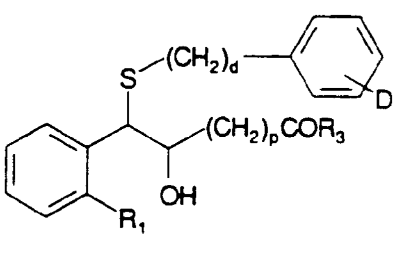

(XVII)

dans laquelle d, X, $R_1$ et p sont tels que définis plus haut, avec un alcanol.

**11.** Procédé suivant la revendication 10B, dans lequel le composé contenant la fonction mercapto est :

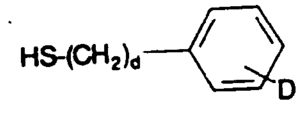

dans laquelle d est 0 ou 1 et D est tel que défini plus haut, et le tétraalcoolate de titane est l'isopropylate de titane.

**Revendications pour les Etats contractants suivants : ES, GR**

1. Procédé pour la préparation d'un composé de formule (I) ou d'un sel de celui-ci acceptable du point de vue pharmaceutique :

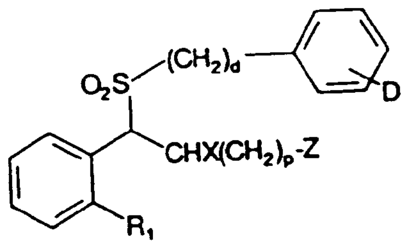

(I)

dans laquelle $R_1$ est un groupe 8-phényloctyle, X est un groupe OH ou alcoxy en $C_{1-4}$, d est 0 ou 1, p est 0 ou 1, Z est $COR_3$ et D est $COR_3$ dans lesquels $R_3$ est un groupe OH ou alcoxy en $C_{1-6}$, qui comprend l'oxydation de l'atome de soufre d'un composé de formule (XVIII) :

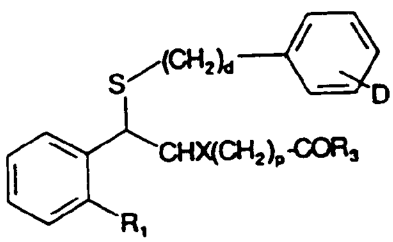

(XVIII)

dans laquelle X est un groupe OH ou alcoxy en $C_{1-4}$, et $R_1$, $R_3$, d et p sont tels que définis dans la formule (I), et la formation éventuelle d'un sel acceptable du point de vue pharmaceutique.

2. Procédé suivant la revendication 1, dans lequel p est 0 et X est un groupe méthoxy.

3. Procédé suivant la revendication 2, dans lequel le composé préparé est l'acide [R-(R*,S*)]-3-[(4-carboxyphényl)sulfonyl]-2-méthoxy-3-[2-(8-phényloctyl)-phényl]-propionique ou un sel de celui-ci acceptable du point de vue pharmaceutique.

4. Procédé la préparation d'un composé de formule (II) ou d'un sel de celui-ci acceptable du point de vue pharmaceutique :

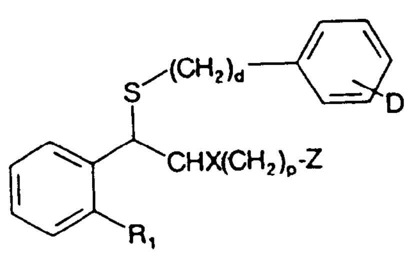

(II)

dans laquelle $R_1$ est un groupe 8-phényloctyle, X est un groupe OH ou alcoxy en $C_{1-4}$, d est 0 ou 1, p est 0 ou 1, Z est $COR_3$ et D est $COR_3$ dans lesquels $R_3$ est un groupe OH ou alcoxy en $C_{1-6}$, qui comprend :
A) la réaction d'une base forte avec un composé de formule (XIV) :

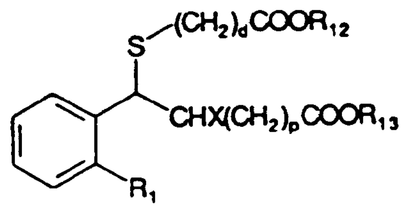

(XIV)

dans laquelle d est 1, X est un groupe OH, $R_1$ et p sont tels que définis dans la revendication 4 et $R_{12}$ et $R_{13}$ sont indépendamment un groupe alkyle en $C_{1-4}$ pour fournir un thiol de formule (XV) :

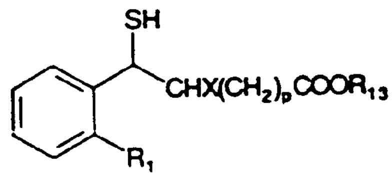

(XV)

dans laquelle X, $R_1$ et p sont tels que définis plus haut dans la formule (XIV) et la réaction du thiol de formule (XV) avec un agent alkylant pour fournir un composé de formule (I); ou
B) la réaction d'un époxyde de formule (XVI) :

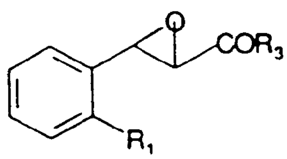

(XVI)

avec un composé HS-R en présence de tétraalcoolate de titane pour obtenir un composé de formule (I) dans laquelle X est un groupe OH; ou
C) l'éthérification d'un composé de formule (XVII) :

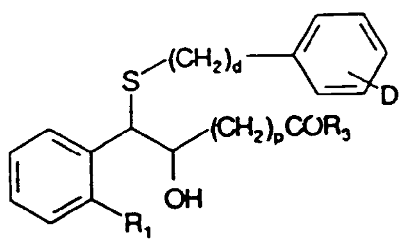

(XVII)

dans laquelle d, X, $R_1$ et p sont tels que définis plus haut, avec un alcanol.

5. Procédé suivant la revendication 4B, dans lequel le composé contenant la fonction mercapto est :

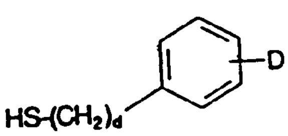

dans laquelle d est 0 ou 1 et D est tel que défini plus haut, et le tétraalcoolate de titane est l'isopropylate de titane.

6. Procédé suivant les revendications 4 ou 5, dans lequel le composé préparé est :
l'acide 2-hydroxy-3-[(5-carboxy-2-méthoxyphénylméthyl)-thio]-3-[2-(8-phényloctyl)-phényl]-propionique;
l'acide [R-(R*,S*)]-3-[(4-carboxyphényl)-thio]-2-méthoxy-3-[2-(8-phényloctyl)-phényl]-propionique;
l'acide [R-(R*,S*)]-3-[(4-carboxyphényl)-thio]-2-hydroxy-3-[2-(8-phényloctyl)-phényl]-propionique;
l'acide [R-(R*,S*)]-3-[(4-carboxy-2-méthoxyphénylméthyl)-thio]-2-hydroxy-3-[2-(8-phényloctyl)-phényl]-propionique;
l'acide [R-(R*,S*)]-3-[(4-carboxy-2-fluorophénylméthyl)-thio]-2-hydroxy-3-[2-(8-phényloctyl)-phényl]-propionique;
l'acide [R-(R*,S*)]-3-[(4-carboxyméthylphényl)-thio]-2-hydroxy-3-[2-(8-phényloctyl)-phényl]-propionique;
ou un sel de ceux-ci acceptable du point de vue pharmaceutique.

7. Procédé pour la préparation d'une composition pharmaceutique, qui comprend la mise en association d'un composé de formule (I) ou d'un sel de celui-ci acceptable du point de vue pharmaceutique telle que définie dans la revendication 1, et d'un support acceptable du point de vue pharmaceutique.

8. Utilisation d'un composé de formule (I) telle que définie dans la revendication 1, dans la fabrication d'un médicament pour le traitement de l'asthme.

9. Procédé pour la préparation d'une composition pharmaceutique, qui comprend la mise en association d'un composé de formule (II) ou d'un sel de celui-ci acceptable du point de vue pharmaceutique elle que définie dans la revendication 4, et d'un support acceptable du point de vue pharmaceutique.

10. Utilisation d'un composé de formule (II) telle que définie dans la revendication 4, dans la fabrication d'un médicament pour le traitement de l'asthme.